(19) Europäisches Patentamt
European Patent Office
Office européen des brevets

(11) Veröffentlichungsnummer : **0 056 577**
**B1**

(12) # EUROPÄISCHE PATENTSCHRIFT

(45) Veröffentlichungstag der Patentschrift : **19.12.84**

(21) Anmeldenummer : **82100040.3**

(22) Anmeldetag : **07.01.82**

(51) Int. Cl.³ : **C 07 D405/14**, C 09 B 57/02, D 06 L 3/12, H 01 S 3/16 // (C07D405/14, 249/06, 311/16, 249/08)

(54) **Triazolylcumarinverbindungen, sowie deren Herstellung und Verwendung als optische Aufheller, Scintillatoren und Laserfarbstoffe.**

(30) Priorität : **16.01.81 DE 3101141**

(43) Veröffentlichungstag der Anmeldung : **28.07.82 Patentblatt 82/30**

(45) Bekanntmachung des Hinweises auf die Patenterteilung : **19.12.84 Patentblatt 84/51**

(84) Benannte Vertragsstaaten : **CH DE FR GB LI**

(56) Entgegenhaltungen :
**EP-A- 0 021 304**
**AT-B- 284 834**
**AT-B- 311 295**
**DE-A- 2 844 299**
**Die Akte enthält technische Angaben, die nach dem Eingang der Anmeldung eingereicht wurden und die nicht in dieser Patentschrift enthalten sind.**

(73) Patentinhaber : **BAYER AG**
**Konzernverwaltung RP Patentabteilung**
**D-5090 Leverkusen 1 Bayerwerk (DE)**

(72) Erfinder : **Berneth, Horst, Dr.**
**Bergstrasse 47**
**D-5068 Odenthal-Glöbusch (DE)**
Erfinder : **Raue, Roderich, Dr.**
**Berta-von-Suttner-Strasse 48**
**D-5090 Leverkusen (DE)**

**EP 0 056 577 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Gegenstand der Erfindung sind Triazolylcumarine der Formel

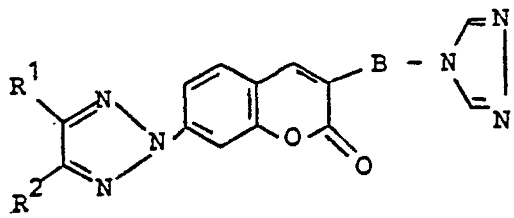

(I)

worin

B eine direkte Bindung oder den 1,4-Phenylenrest und

$R_1$ $C_1$-$C_4$-Alkyl

$R_2$ $C_1$-$C_4$-Alkyl oder gegebenenfalls durch Phenyl, $CH_3$, $OCH_3$ oder Cl substituiertes Phenyl bedeuten oder

$R_1$ und $R_2$ gemeinsam einen anellierten Benzol- oder Naphthalinring bilden,

sowie deren Quaternierungs- und Protonierungsprodukte.

Geeignete Quaternierungs- und Protonierungsprodukte sind solche, die man aus den « neutralen » Verbindungen der Formel I durch Behandlung mit in der Farbstoff- oder Aufheller-Chemie üblichen Quaternierungs- und Protonierungsmitteln, wie zie z. B. in der DE-OS 2 821 116 oder US-PS 4 051 117 beschrieben sind, erhält.

Bevorzugte Mittel dieser Art sind Dimethylsulfat, Diethylsulfat, Chlorwasserstoffsäure, gegebenenfalls durch Cl oder $CH_3$ substituierte Benzolsulfonsäure Ameisensäure, Essigsäure und Milchsäure.

Bevorzugte erfindungsgemäße Verbindungen sind solche der Formel I, worin

$R_1$ Methyl,

$R_2$ Ethyl, Phenyl oder 4-Biphenylyl bedeuten oder

$R_1$ und $R_2$ gemeinsam die fehlenden Glieder eines über $C_1/C_2$-verknüpften Naphthalinringes bilden.

Die neuen Triazolylcumarine sind nach verschiedenen Verfahren zugänglich.

Ein Verfahren ist dadurch gekennzeichnet, daß man Salicylaldehyde der Formel

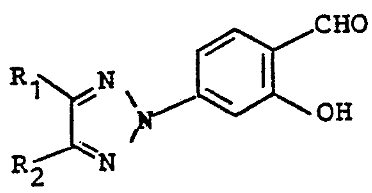

(II)

mit Triazolverbindungen der Formel

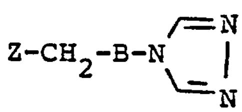

(III)

bzw. deren Quaternierungs -oder Protonierungsprodukten, worin Z eine gegebenenfalls funktionell abgewandelte Carboxylgruppe ist,

in an sich bekannter Weise kondensiert (vgl. US-PS 3 271 412 bzw. DE-PS 1 296 121).

Geeignete Reste Z sind COOH, $COOC_nH_{2n+1}$ (n = 1-4) und CN.

Die Verbindungen der Formel II sind praktisch durchweg bekannt (vgl. DE-OS 2 335 218 und DE-OS 2 848 670).

Auch die « neutralen » Verbindungen der Formel III sind in der Literatur beschrieben worden (vgl. US-PS 4 120 864).

Die « neutralen » Verbindungen der Formel III sind, wenn B eine direkte Bindung bedeutet, auch durch Umsetzung von 1,2,4-Triazolen der Formel

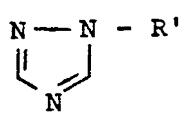

(IV)

worin R' eine Tri-($C_1$-$C_4$-alkyl)silyl-, eine $C_1$-$C_6$-Alkanoyl-, eine Benzoyl-, eine $C_1$-$C_6$-Alkylsulfonyl-, eine

Phenylsufonyl-, eine $C_1$-$C_6$-Alkoxycarbonyl-, eine Phenoxycarbonyl-, eine $C_1$-$C_6$-Alkylaminocarbonyl-, eine Phenylaminocarbonyl-, eine $C_1$-$C_6$-Alkylaminothiocarbonyl- oder eine Phenylaminothiocarbonyl-Gruppe, in der der Phenylrest noch Methyl, Methoxy, Chlor und/oder Hydroxycarbonyl tragen kann, bedeutet, mit vorzugsweise Halogenessigsäurederivaten der Formel

$$Z—CH_2—Hal \qquad (V)$$

worin Hal Chlor, Brom oder Jod und Z eine gegebenenfalls funktionell abgewandelte Carboxylgruppe ist, zugänglich.

Die Quaternierungsprodukte der Verbindungen der Formel III lassen sich, wenn B eine direkte Bindung bedeutet, außer durch Quaternierung der entsprechenden neutralen Verbindungen auch durch Umsetzung von 1,2,4-Triazolen der Formel IV,
worin R' die Quaternierungsgruppe bedeutet, mit vorzugsweise Halogenessigsäurederivaten der Formel V herstellen.

Ein weiteres Verfahren zur Herstellung der Verbindungen der Formel I ist dadurch gekennzeichnet, daß man Aminocumarine der Formel

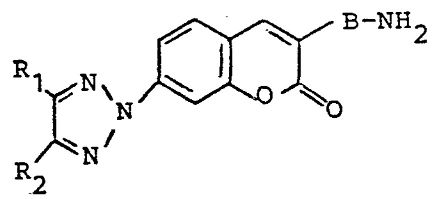

(VI)

mit Amidrazonen der Formel

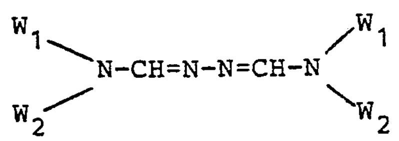

(VII)

worin
$W_1$ und $W_2$ $C_1$-$C_4$-Alkyl, Phenyl oder gemeinsam $—(CH_2)_4—$ oder $—(CH_2)_5—$ bedeuten, in ebenfalls an sich bekannter Weise umsetzt (vgl. US-PS 4 120 864 sowie J. Chem. Soc. *1967*, 1664-1666) und die Reaktionsprodukte gegebenenfalls anschließend quaterniert oder protoniert.

Die Umsetzung von VI mit VII erfolgt vorzugsweise in einem üblichen polaren organischen Lösungsmittel bei Rückflußtemperaturen des Lösungsmittels und gegebenenfalls in Anwesenheit eines sauren oder basischen Katalysators.

Die Verbindungen der Formel VII sind in der vorstehend genannten Literatur beschrieben.

Die Aminocumarine VI sind entweder aus J. Ind. Chem. Soc. *48*, 371 (1971) und US-PS 2 702 296 bekannt oder nach analogen Verfahren erhältlich.

Weiterhin werden die neuen Cumarine der Formel I durch Umsetzung von Amidinocumarinen der Formel

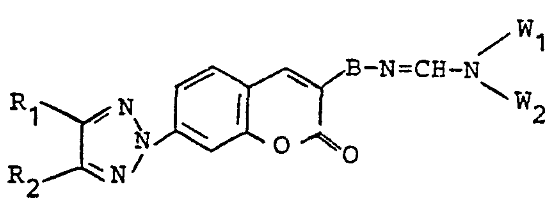

(VIII)

mit Formylhydrazin und gegebenenfalls anschließende Quaternierung bzw. Protonierung erhalten.

Die Verbindungen der Formel VIII erhält man durch Umsetzung von Aminocumarinen der Formel VI oder deren N-Acylierungsprodukten mit Vilsmeyer-Verbindungen der Formel

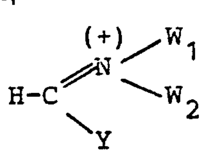

(−)
Anion

(IX)

worin Y eine Abgangsgruppe, vorzugsweise Halogen, bedeutet.

Die Umsetzung der Amidinocumarine (VIII) erfolgt unter ähnlichen Bedingungen wie bei der Umsetzung von VI mit VII.

Schließlich können die Verbindungen der Formel I dadurch hergestellt werden, daß man in Cumarine der Formel

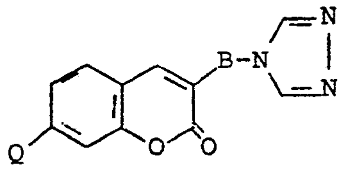

(X)

worin Q für $NH_2$ oder $NH\text{-}NH_2$ steht, in an sich bekannter Weise den 1,2,3-Triazol-2-yl-rest in 7-Stellung Heinführt (vgl. US-PS 3 646 052 sowie DE-OS 1 670 914 = US-PS 3 666 758).

Bevorzugt ist die Umsetzung entsprechender 7-Hydrazinocumarine mit geeigneten α-Oximinoketonen. Die als Ausgangsmaterial benötigten Verbindungen der Formel X erhält man in üblicher Weise (vgl. z. B. DE-OS 2 528 698) durch Umsetzung von entsprechenden Acylaminosalicylaldehyden mit den Verbindungen III, anschließende Abspaltung der Acylschutzgruppe und gegebenenfalls Umwandlung der Aminogruppe in eine Hydrazinogruppe (vgl. DE-A 2 528 698 = US-A 4 069 228 und DE-A 1 94 845 = US-A 3 646 052).

Die neuen Cumarinverbindungen der Formel I unterscheiden sich von den Fluoreszenzfarbstoffen gemäß EP-A1-0 021 304 durch das Fehlen einer CN-Gruppe in 4-Stellung und ebenso wie von den optischen Aufhellern gemäß AT-B 2 848 34, AT-B 311 295 und DE-A 2 844 299 durch die spezielle Auswahl der Triazolylreste in 3- und 7-Stellung. Sie zeigen in gelöstem oder feinverteiltem Zustand eine starke Fluoreszenz und eignen sich hervorragend als Scintillatoren, Laserfarbstoffe und vorzugsweise als optische Aufheller. Während die « neutralen » Verbindungen bevorzugt zum Weißtönen von Textilmaterialien aus hydrophoben Fasern, insbesondere Polyesterfasern eingesetzt werden, dienen die quaternierten und protonierten Produkte zum Aufhellen von sauer-modifizierten Fasermaterialien, wie z. B. Acrylfasern nach üblichen Färbemethoden.

Die neuen Weißtöner zeichnen sich dabei durch hohe Farbstärkte, gutes Aufbauvermögen und eine geringe Neigung zum Vergrünen aus — selbst bei hohen Wirkstoffkonzentrationen.

Beispiel 1

6,50 g (20 mmol) 3-(Dimethylaminomethylenamino)-7-(4-ethyl-5-methyl-2H-1,2,3-triazol-2-yl) cumarin und
1,44 g (24,0 mmol) Formylhydrazin werden in
18 ml wasserfreiem Dimethylformamid 1,5 h unter $N_2$-Atmosphäre refluxiert. Nach dem Abkühlen wird der dicke gelbe Brei scharf abgesaugt und mit
10 ml wasserfreiem Dimethylformamid und
20 ml Chloroform gewaschen und getrocknet : 2,00 g (31 %) blaßgelbes Pulver, das aus Dimethylacetamid umkristallisiert wird : 1,62 g (25 %) 3-(1,2,4-Triazol-4-yl)-7-(4-ethyl-5-methyl-2H-1,2,3-triazol-2-yl)-cumarin in Form blaßgelber verfilzter Nädelchen vom Schmp. 263-264 °C.

IR (KBr) : 3 110, 1 740, 1 618 $cm^{-1}$.
UV (DMF) : $\lambda_{max}$ (lg ε) = 347 nm (4.518).
Fluoreszenz : rotstichig blau.

Die Ausgangsmaterialien können auf folgendem Weg hergestellt werden :

50,6 g (200 mmol) 2-Hydroxy-4-(4-ethyl-5-methyl-2H-1,2,3-triazol-2-yl)-benzaldehyd-Na-Salz,
6,00 g (80,0 mmol) wasserfreies Natriumacetat,
18,0 g (240 mmol) Aminoessigsäure und
102 g (1,00 mol) Acetanhydrid werden während 1 h unter $N_2$-Atmosphäre zum Rückfluß gebracht und 15 h am Rückfluß gekocht. Nach Abkühlen auf 90 °C werden
100 ml Methanol zugesetzt, 30 Min. refluxiert, auf 0 °C gekühlt, abgesaugt, mit
350 ml Methanol gewaschen und getrocknet : 55,3 g (89 %) braunes Pulver, das aus 1,2-Dichlorbenzol umkristallisiert wird : 19,6 g (31 %) 3-Acetylamino-7-(4-ethyl-5-methyl-2H-1,2,3-triazol-2-yl)cumarin in Form rosa Schuppen vom Schmp. 237-238 °C.
15,6 g (50,0 mmol) 3-Acetylamino-7-(4-ethyl-5-methyl-2H-1,2,3-triazol-2-yl)-cumarin werden in
100 ml wasserfreiem Dimethylformamid suspendiert. Bei 10-15 °C werden
11,5 g (75,0 mmol) Phosphoroxychlorid zugetropft und 22 h bei Raumtemperatur gerührt. Der Brei wird in
500 ml Eiswasser eingetragen, filtriert und mit Natriumcarbonat auf pH = 8 gebracht. Das gelbe

Kristallisat wird abgesaugt, mit Wasser gewaschen und getrocknet : 15,5 g (95,5 %). Umkristallisation aus Ethanol ergibt 12,9 g (80 %) 3-(Dimethylaminomethylenamino)-7-(4-ethyl-5-methyl-2H-1,2,3-triazol-2-yl) cumarin in Form hellgelber Kriställchen vom Schmp. 158-159 °C.

IR (KBr) : 1 723, 1 620 $cm^{-1}$.
UV (DMF) : $\lambda_{max}$ (lg ε) = 280 (3.975), 370 nm (4.615).

Auf diesem Weg lassen sich in analoger Weise die Verbindungen der Beispiele 2-8 erhalten. In den Beispielen 5-8 ist die Aminoessigsäure durch 4-Aminophenylessigsäure zu ersetzen.

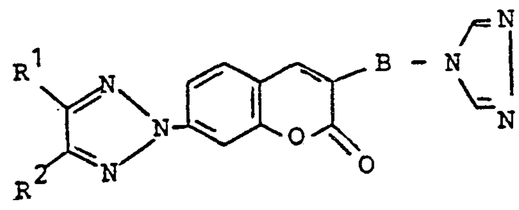

| Beispiel | $R^1$ | $R^2$ | B | Fluoreszenz |
|---|---|---|---|---|
| 2 | $CH_3$ | $C_6H_5$ | - | blau |
| 3 | $C_2H_5$ | 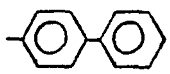 | - | blau |
| 4 | 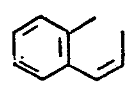 | | - | grünstichig-blau |
| 5 | $CH_3$ | $C_2H_5$ | 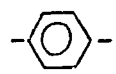 | blau |
| 6 | $CH_3$ | $C_6H_5$ | 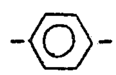 | grünstichig-blau |
| 7 | $C_2H_5$ | 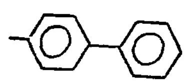 | 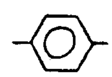 | " |
| 8 | 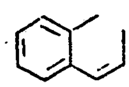 | | 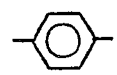 | " |

Beispiel 9

15,0 g (50,0 mmol) 2-Hydroxy-4-(4-methyl-5-phenyl-2H-1,2,3-triazol-2-yl)-benzaldehyd-Na-Salz,
7,75 g (50,0 mmol) 1,2,4-Triazol-4-yl-essigsäureethylester,
1,50 g (25,0 mmol) Eisessig und
500 mg (6,00 mmol) Piperidin werden unter $N_2$-Atmosphäre in
70 ml wasserfreiem Ethanol 8 h refluxiert. Es wird scharf abgesaugt und mit Ethanol und Wasser gewaschen. Schließlich wird nochmal mit
100 ml Ethanol 2 h bei 60 °C verrührt, abgesaugt und getrocknet : 12,0 g (65 %) hellgelbes Kristallpulver. Umkristallisation aus Dimethylformamid ergibt 9,64 g (52 %) 3-(1,2,4-Triazol-4-yl)-7-(4-methyl-5-phenyl 2H-1,2,3-triazol-2-yl)-cumarin in Form hellgelber Mikroplättchen vom Schmp. 324-326 °C (Zers.)

IR (KBr) : 3 110, 1 745, 1 733, 1 618 $cm^{-1}$.
UV (DMF) : $\lambda_{max}$ (lg ε) = 355 nm (4.617).
Fluoreszenz : blau

Die Verbindung ist identisch mit der des Beispiels 2.
Auf diesem Weg lassen sich in analoger Weise die Verbindungen der Beispiele 10-16 herstellen. Statt des 1,2,4-Triazol-4-yl-essigsäureethylester kann auch der Ester anderer Alkohole verwendet werden wie

Methyl-, Propyl-, Butyl- oder Benzylester. In den Beispielen 13-16 ist statt dessen ein Ester der 4-(1,2,4-Triazol-4-yl)-phenylessigsäure, beispielsweise der Ethylester, zu verwenden.

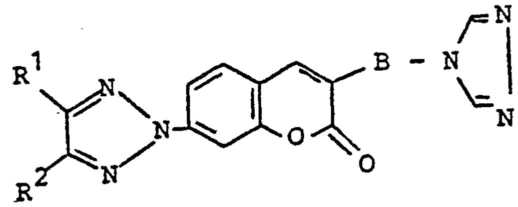

| Beispiel | $R^1$ | $R^2$ | B | Fluoreszenz | identisch mit Beisp. |
|---|---|---|---|---|---|
| 10 | $CH_3$ | $C_2H_5$- | - | rotstichig blau | 1 |
| 11 | $C_2H_5$ | 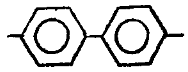 | - | blau | 3 |
| 12 | 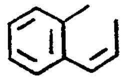 | | - | grünstichig blau | 4 |
| 13 | $CH_3$ | $C_2H_5$- | 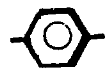 | blau | 5 |
| 14 | $CH_3$ | $C_6H_5$- | 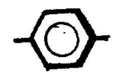 | grünstichig blau | 6 |
| 15 | $C_2H_5$ | 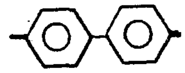 | 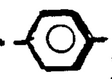 | grünstichig blau | 7 |
| 16 | 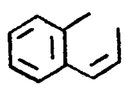 | | 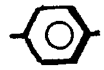 | grünstichig blau | 8 |

Beispiel 17

17,3 g (50,0 mmol) 3-(4-Aminophenyl)-7-(4-ethyl-5-methyl-2H-1,2,3-triazol-2-yl)cumarin,
10,5 g (75,0 mmol) 1,2-Bis(dimethylaminomethylen)hydrazin und
200 mg 4-Methylbenzolsulfonsäure werden in
40 ml wasserfreiem Dimethylformamid 10 h unter $N_2$-Atmosphäre refluxiert. Dann werden nochmal
3,50 g (25,0 mmol) 1,2-Bis(dimethylaminomethylen)-hydrazin zugesetzt und weitere 10 h refluxiert. Beim Abkühlen scheiden sich 16,1 g (81 %) hellgelbes Kristallisat aus. Umkristallisation aus Dimethylformamid ergibt 14,3 g (72 %) 3-(4-(1,2,4-Triazol-4-yl)-phenyl)-7-(4-ethyl-5-methyl-2H-1,2,3-triazol-2-yl)-cumarin in Form blaßgelber Kriställchen vom Schmp. 321-323 °C.

IR (KBr) : 3 110, 1 730, 1 615, 1 532 $cm^{-1}$.
UV (DMF) : $\lambda_{max}$ (lg ε) = 357 nm (4.611).
$^1$H-NMR([$D_6$]DMSO) : δ 1,24 (t, J = 7,5 Hz ; 3H, $CH_3CH_2$), 2,30 (s ; 3H, $CH_3$), 2,67 (q, J = 7,5 Hz ; 2H ; $CH_3CH_2$) 7,84 (mc ; 7H), 8,31 (s ; 1H, 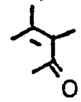 ), 9,13 (s ; 2H, Triazol).

Fluoreszenz : blau

Die Verbindung ist identisch mit der der Beispiele 5 und 13.
Die Ausgangsmaterialien können auf folgendem Weg hergestellt werden :

50,6 g (200 mmol) 2-Hydroxy-4-(4-ethyl-5-methyl-2H-1,2,3-triazol-2-yl)benzaldehyd-Na-Salz,
41,8 g (200 mmol) 4-Nitrophenylessigsäureethylester und
2 ml Piperidin werden in
250 ml Ethanol 4 h refluxiert. Nach Abkühlen wird mit Eisessig neutralisiert, mit Ethanol und Wasser gewaschen und getrocknet : 46,3 g (61,5 %). Umkristallisation aus Toluol liefert 41,4 g (55 %) 3-(4-Nitrophenyl)-7-(4-ethyl-5-methyl-2H-1,2,3-triazol-2-yl) cumarin in Form eines hellgelben Kristallpulvers vom Schmp. 278-279 °C.

IR (KBr) : 1 723, 1 618, 1 516, 1 350 $cm^{-1}$.

37,6 g (100 mmol) 3-(4-Nitrophenyl)-7-(4-ethyl-5-methyl-2H-1,2,3-triazol-2-yl)-cumarin werden in
1000 ml Ethanol suspendiert. In der Siedehitze wird eine Lösung von
160 g (708 mmol) Zinn(II)chlorid in
125 ml konz. Salzsäure während 1,5 h zugetropft und weitere 3 h refluxiert. Nach dem Abkühlen wird abgesaugt, mit Ethanol gewaschen und der Filterkuchen durch Verrühren mit
30 g Kaliumcarbonat in
600 ml Wasser und
600 ml Chloroform deprotoniert. Die Chloroformphase wird nach dem Trocknen über Kaliumcarbonat eingedampft und der Rückstand aus Toluol umkristallisiert : 29,5 g (85 %) 3-(4-Aminophenyl)-7-(4-ethyl-5-methyl-2H-1,2,3-triazol-2-yl)-cumarin in Form eines hellgelben Pulvers vom Schmp. 162-163 °C.

IR (KBr) : 3 465, 3 375, 1 723, 1 620 $cm^{-1}$.
UV (DMF) : $\lambda_{max}$ (lg ε) = 306 (4.204), 392 nm (4.453).

Auf diesem Weg lassen sich in analoger Weise die Verbindungen der Beispiele 18-20 herstellen.

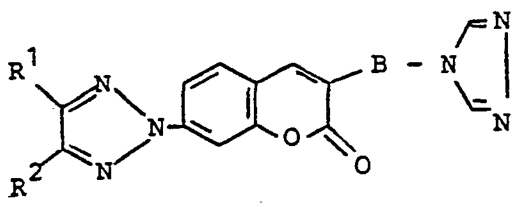

| Beispiel | $R^1$ | $R^2$ | Fluoreszenz | identisch mit Beispiel |
|---|---|---|---|---|
| 18 | $CH_3$ | $C_6H_5$ | grünstichig-blau | 6, 14 |
| 19 | $C_2H_5$ | 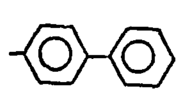 | grünstichig-blau | 7, 15 |
| 20 | | 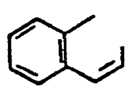 | grünstichig-blau | 8, 16 |

Beispiel 21

Zu
6,02 g (20,0 mmol) 2-Hydroxy-4-(4-methyl-5-phenyl-2H-1,2,3-triazol-2-yl)-benzaldehyd-Na-Salz und
4,80 g (80,0 mmol) Eisessig in
20 ml wasserfreiem Dimethylformamid werden unter $N_2$-Atmosphäre
3,48 g (22,0 mmol) 1-Methyl-4-cyanomethyl-1,2,4-triazolium-chlorid gegeben und 24 h bei Raumtemperatur gerührt. Der Niederschlag wird abgesaugt, mit Dimethylformamid gewaschend und getrocknet : 3,60 g (43 %) hellgelbes Pulver. Umkristallisation aus Wasser liefert 2,90 g (35 %) 1-Methyl-4-[7-(4-methyl-5-phenyl-2H-1,2,3-triazol-2-yl)-cumarin-3-yl]-1,2,4-triazolium-chlorid in Form hellgelber Kriställchen vom Schmp. 249-252 °C (Zers.).

IR (KBr) : 3 060, 1 737, 1 620 $cm^{-1}$.
UV (DMF) : $\lambda_{max}$ (lg ε) = 360 nm (4.577).
Fluoreszenz : blau.

Die Reaktion läßt sich in analoger Weise auch mit z. B. 1-Methyl-4-(methylcarbonylmethyl)-1,2,4-triazoliumchlorid oder 1-Methyl-4-(ethoxycarbonylmethyl)-1,2,4-triazoliumchlorid durchführen.
Die Ausgangsmaterialien können auf folgendem Weg hergestellt werden :

16,6 g (200 mmol) 1-Methyl-1,2,4-triazol und
15,1 g (200 mmol) Chloracetonitril werden 24 h auf 80 °C erwärmt. Beim Abkühlen kristallisiert die Schmelze spontan durch : 31,7 g (100 %) farblose Nadeln. Umkristallisation aus Ethanol/Wasser ergibt

28,8 g (91 %) 1-Methyl-4-cyanomethyl-1,2,4-triazoliumchlorid in Form farbl. Nadeln vom Schmp. 186-187 °C.

IR (KBr) : 3 170, 2 255, 1 820, 1 583 $cm^{-1}$.

Analog können beispielsweise auch 1-Ethyl-, 1-Benzyl-, 1-(2-Hydroxyethyl)- oder 1-Phenyl-1,2,4-triazol mit beispielsweise Bromessigsäure, Chloressigsäuremethylester oder Bromessigsäureethylester umgesetzt werden.

Auf diesem Weg lassen sich in analoger Weise die Verbindungen der Beispiele 22-27 herstellen.

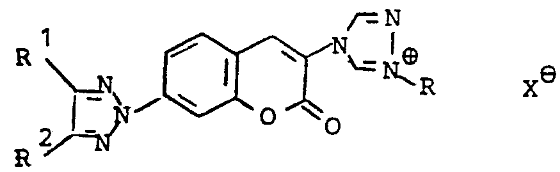

| Beispiel | $R^1$ | $R^2$ | R | X | Fluoreszenz |
|---|---|---|---|---|---|
| 22 | $CH_3$ | $C_2H_5$ | $CH_3$ | Br | rotstichig blau |
| 23 | $CH_3$ | $C_6H_5$ | $C_2H_5$ | Cl | blau |
| 24 | 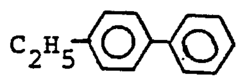 | | 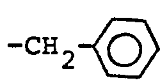 | Br | blau |
| 25 | 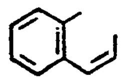 | | $-CH_2CH_2OH$ | Cl | grünstichig blau |
| 26 | $CH_3$ | $C_2H_5$ | $-CH_2COOH$ | Cl | blau |
| 27 | $C_2H_5$ | $C_6H_5$ | $C_6H_5$ | Cl | grünstichig blau |

Beispiel 28

7,96 g (20,0 mmol) 3-(4-(1,2,4-Triazol-4-yl)-phenyl)-7-(4-ethyl-5-methyl-2H-1,2,3-triazol-2-yl)-cumarin werden in

45 ml wasserfreiem Dimethylformamid bei 100 °C unter $N_2$-Atmosphäre mit

2,77 g (22,0 mmol) Dimethylsulfat versetzt. Es entsteht eine Lösung, die abgekühlt und mit Toluol bis zur vollständigen Fällung versetzt wird : 8,16 g (78 %) 1-Methyl-4-[4-(7-(4-ethyl-5-methyl-2H-1,2,3-triazol-2-yl)cumarin-3-yl)-phenyl]-1,2,4-triazolium-methosulfat in Form blaßgelber Kriställchen vom Schmp. 221-225 °C (Zers.).

IR (KBr) : 1 724, 1 620 $cm^{-1}$.
UV (DMF) : $\lambda_{max}$ (lg $\varepsilon$) = 356 nm (4.607)
$^1$H-NMR ([$D_6$]DMSO) : $\delta$ = 1,26 (t, J = 7,5 Hz ; 3H, $C\underline{H}_3CH_2$), 2,33 (s ; 3H, $CH_3$), 2,73 (q, J = 7,5 Hz ; 2H, $CH_3C\underline{H}_2$), 3,42 (s ; 3H, $CH_3OSO_3$), 4,21 (s ; 3H, $CH_3N^{\oplus}$), 7,8-8,15 (m ; 7H), 8,45 (s ; 1H, H 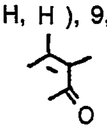 ), 9,83 (s ; 1H, Triazol), 10,85 (s ; 1H, Triazol).
Fluoreszenz : blau

Auf diesem Weg lassen sich in analoger Weise die Verbindungen der Beispiele 29-35 herstellen (bei den Beispielen 31 und 33 werden substituierte Alkylhalogenide verwendet).

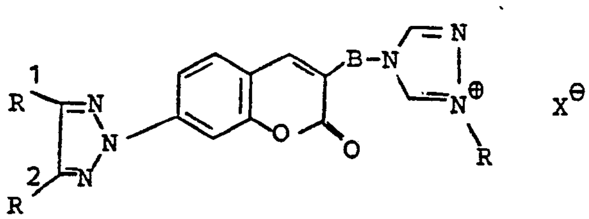

| Beispiel | $R^1$ | $R^2$ | B | R | X | Fluoreszenz |
|---|---|---|---|---|---|---|
| 29 | $CH_3$ | $C_2H_5$ | - | $CH_3$ | $CH_3OSO_3$ | rotstichig blau |
| 30 | $CH_3$ | $C_6H_5$ | - | $C_2H_5$ | $C_2H_5OSO_3$ | blau |
| 31 | $C_2H_5$ | 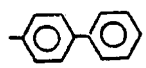 | - | $C_3H_7$ | Br | blau |
| 32 | 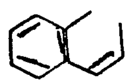 | | - | $CH_3$ | $CH_3OSO_3$ | grünstichig blau |
| 33 | $CH_3$ | $C_6H_5$ | 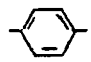 | Benzyl | Br | grünstichig blau |
| 34 | $C_2H_5$ | 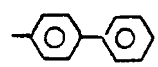 | 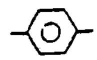 | $CH_3$ | $CH_3OSO_3$ | grünstichig blau |
| 35 | 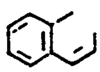 | | 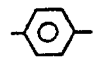 | $-CH_2$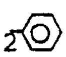 | 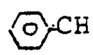$CH_2OSO_3$ | grünstichig blau |

Beispiel 36

3,70 g (10,0 mmol) 3-(1,2,4-Triazol-4-yl)-7-(4-methyl-5-phenyl-2H-1,2,3-triazol-2-yl)cumarin und 3,45 g (20,0 mmol) Benzolsulfonsäuremethylester werden in 25 ml wasserfreiem 1,2-Dichlorbenzol 10 min auf 190 °C erhitzt. Die Suspension wird nach dem Abkühlen abgesaugt, mit 5 ml heißem 1,2-Dichlorbenzol gewaschen und getrocknet: 5,42 g (100 %) 1-Methyl-4-[7-(4-methyl-5-phenyl-2H-1,2,3-triazol-2-yl)cumarin-3-yl]-1,2,4-triazolium-benzosulfonat in Form eines hellgelben Pulvers vom Schmp. 261-262° (Zers.)

IR (KBr): 3 060, 1 737, 1 620 $cm^{-1}$.
UV (DMF): $\lambda_{max}$ (lg ε) = 360 nm (4.577).
Fluoreszenz: blau

Auf diesen Weg lassen sich in analoger Weise die Verbindungen der Beispiele 37-43 herstellen.

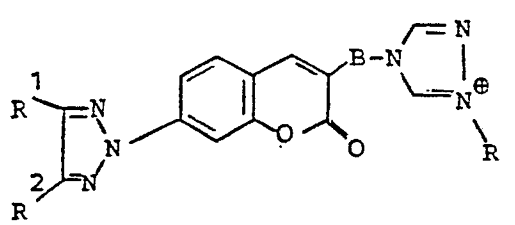

$X^{\ominus}$

| Beispiel | $R^1$ | $R^2$ | B | R | X | Fluoreszenz |
|---|---|---|---|---|---|---|
| 37 | $CH_3$ | $C_2H_5$ | - | $-CH_2$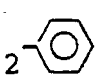 | 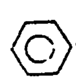$-SO_3$ | rotstichig blau |
| 38 | $C_2H_5$ | 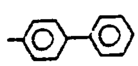 | - | $CH_3$ | $CH_3SO_3$ | blau |
| 39 | 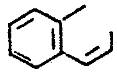 | | - | $C_2H_5$ | $CH_3$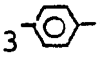$-SO_3$ | grünstichig blau |

(Fortsetzung)

| Beispiel | $R^1$ | $R^2$ | B | R | X | Fluoreszenz |
|---|---|---|---|---|---|---|
| 40 | $CH_3$ | $C_2H_5$ | 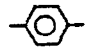 | $CH_3$ | $CH_3SO_3$ | blau |
| 41 | $CH_3$ | $C_6H_5$ | 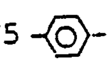 | $C_2H_5$ | Cl-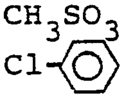-$SO_3$ | grünstichig blau |
| 42 | $C_2H_5$ | 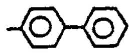 | 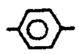 | $CH_3$ | 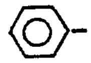-$SO_3$ | grünstichig blau |
| 43 | 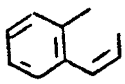 | | 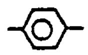 | $CH_3$ | $CH_3SO_3$ | grünstichig blau |

Beispiel 44

6,44 g (20 mmol) 3-(1,2,4-Triazol-4-yl)-7-(44-ethyl-5-methyl-2H-1,2,3-triazol-2-yl)cumarin werden bei 50 °C in
15 g Ameisensäure und
5 g Toluol gelöst. Dann wird solange Ethylenoxid eingeleitet, bis eine von Toluol befreite Probe klar in Wasser löslich ist. Anschließend wird vom ganzen Ansatz das Toluol und der größte Teil der Ameisensäure im Vakuum abdestilliert. Zurück bleiben 1-(2-Hydroxyethyl)-4-[7-(4-ethyl-5-methyl-2H-1,2,3-triazol-2-yl)cumarin-3-yl]-1,2,4-triazolium-formiat und Ameisensäureglycolester, die zu einer 20-30 %igen Lösung in Wasser aufgenommen werden können.
Fluoreszenz : rotstichig blau.

Auf diesem Weg lassen sich in analoger Weise die Verbindungen der Beispiele 45-52 herstellen. Bei Beispiel 52 wird statt Ethylenoxid Acrylnitril zugesetzt.

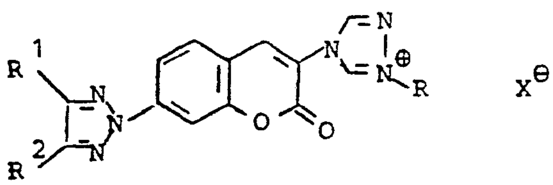

| Beispiel | $R^1$ | $R^2$ | B | R | X | Fluoreszenz |
|---|---|---|---|---|---|---|
| 45 | $CH_3$ | $C_6H_5$ | - | $-CH_2CH_2OH$ | $CH_3COO$ | blau |
| 46 | $C_2H_5$ | 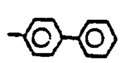 | - | $-CH(CH_3)-CH_2OH$ | HCOO | blau |
| 47 | 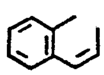 | | - | $-CH_2CH_2OH$ | $CH_3CH(OH)COO$ | grünstichig blau |
| 48 | $CH_3$ | $C_2H_5$ | 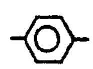 | $-CH_2CH_2OH$ | HCOO | blau |
| 49 | $CH_3$ | $C_6H_5$ | 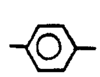 | $-CH(CH_3)-CH_2OH$ | $CH_3COO$ | grünstichig blau |
| 50 | $C_2H_5$ | 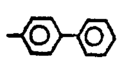 | 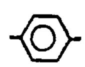 | $-CH(CH_3)-CH_2OH$ | $CH_3CH(OH)COO$ | " |
| 51 | 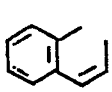 | | 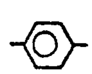 | $-CH_2CH_2OH$ | HCOO | " |
| 52 | $CH_3$ | $C_6H_5$ | - | $-CH_2CH_2CN$ | HCOO | blau |

Beispiel 53

3,98 g (10,0 mmol) 3-(4-(1,2,4-Triazol-4-yl)-phenyl)-7-(4-ethyl-5-methyl-2H-1,2,3-triazol-2-yl)-cumarin werden in
15 ml Dimethylformamid auf 70 °C erwärmt und solange
70 %ige Perchlorsäure zugesetzt, bis eine Lösung entsteht. Nach dem Abkühlen wird mit Wasser verdünnt und der blaßgelbe Niederschlag abgesaugt und mit Wasser gewaschen : 3,43 g (85 %) 4-[4-(7-(4-Ethyl-5-methyl-2H-1,2,3-triazol-2-yl)-cumarin-3-yl)-phenyl]-1,2,4-triazolium-perchlorat vom Schmp. 260-264 °C (Zers.)
Fluoreszenz : blau.

Auf diesem Weg lassen sich in analoger Weise die Verbindungen der Beispiele 54-60 herstellen.

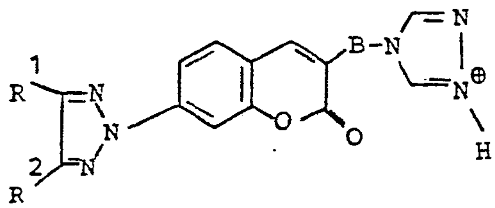 $X^{\ominus}$

| Beispiel | $R^1$ | $R^2$ | B | X | Fluoreszenz |
|---|---|---|---|---|---|
| 54 | $CH_3$ | $C_2H_5$ | - | $BF_4$ | rotstichig blau |
| 55 | $CH_3$ | $C_6H_5$ | - | Cl | blau |
| 56 | $C_2H_5$ | 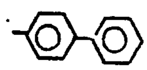 | - | Br | blau |
| 57 | 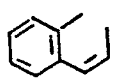 | | - | $ClO_4$ | grünstichig blau |
| 58 | $CH_3$ | $C_6H_5$ | 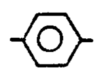 | Cl | " |
| 59 | $C_2H_5$ | 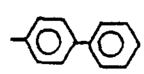 | 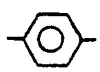 | $HSO_4$ | grünstichig blau |
| 60 | 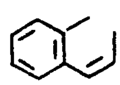 | | 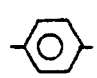 | Br | " |

Beispiel 61

Polyacrylnitril-Textilgewebe werden im Flottenverhältnis 1 : 40 30 Minuten kochend mit einer Färbeflotte behandelt, die 0,2 % der Verbindung der Formel 21, 8 % Na-chorit 50 %ig, 4 % Na-nitrat, 4 % Chloritstabilisator (alle Prozentangaben bezogen auf das Textilmaterial) enthält und mit Ameisensäure auf pH 3,5 gestellt ist. Nach dem Spülen und Trocknen erhält man ein sehr gut und brillant aufgehelltes Polyacrylnitrilgewebe.

Ähnliche Ergebnisse erhält man, wenn man gleich wie oben beschrieben verfährt, jedoch die Verbindungen der Formeln 24, 26, 32, 33, 38 oder 40 einsetzt.

**Ansprüche**

1. Triazolylcumarine der Formel

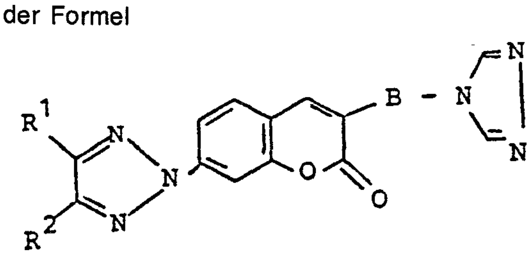

worin

B eine direkte Bindung oder den 1,4-Phenylenrest und

$R_1$ $C_1$-$C_4$-Alkyl

$R_2$ $C_1$-$C_4$-Alkyl oder gegebenenfalls durch Phenyl, $CH_3$, $OCH_3$ oder Cl substituiertes Phenyl bedeuten oder

$R_1$ und $R_2$ gemeinsam einen anellierten Benzol- oder Naphthalinring bilden.

2. Triazolylcumarine gemäß Anspruch 1, dadurch gekennzeichnet daß

$R_1$ Methyl,

$R_2$ Ethyl, Phenyl oder 4-Biphenylyl bedeuten oder

$R_1$ und $R_2$ gemeinsam die fehlenden Glieder eines über $C_1/C_2$-verknüpften Naphthalinringes bilden.

3. Triazolylcumarine gemäß Anspruch 1, dadurch gekennzeichnet, daß B für eine direkte Bindung steht.

4. Triazolylcumarin gemäß Anspruch 1, dadurch gekennzeichnet, daß

$R_1$ Methyl und

$R_2$ Phenyl bedeuten.

5. Verfahren zur Herstellung von Triazolylcumarinen gemäß Anspruch 1, dadurch gekennzeichnet, daß man Salicylaldehyde der Formel

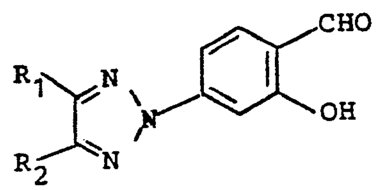

mit Triazolverbindungen der Formel

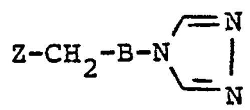

bzw. deren Quaternierungs- oder Protonierungsprodukten, worin Z eine gegebenenfalls funktionell abgewandelte Carboxylgruppe ist, in an sich bekannter Weise kondensiert.

6. Verfahren zur Herstellung von Triazolylcumarinen gemäß Anspruch 1, dadurch gekennzeichnet, daß man Aminocumarine der Formel

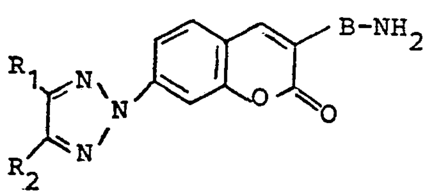

mit Amidrazonen der Formel

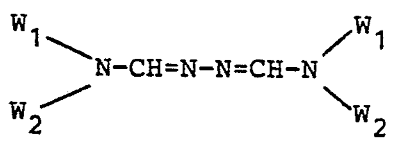

worin

$W_1$ und $W_2$ $C_1$-$C_4$-Alkyl, Phenyl oder gemeinsam $-(CH_2)_4-$ oder $-(CH_2)_5-$ bedeuten, umsetzt und die Reaktionsprodukte gegebenenfalls quaterniert oder protoniert.

7. Verfahren zur Herstellung von Triazolylcumarinen gemäß Anspruch 1, dadurch gekennzeichnet, daß man Amidinocumarine der Formel

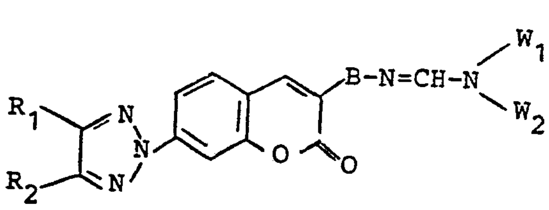

mit Formylhydrazin umsetzt und die Reaktionsprodukte gegebenenfalls quaterniert oder protoniert.

8. Verfahren zur Herstellung von Triazolylcumarinen gemäß Anspruch 1, dadurch gekennzeichnet, daß man Cumarine der Formel

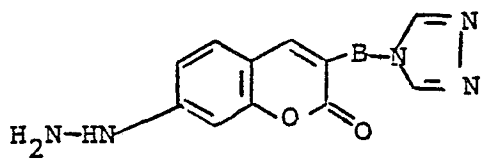

mit entsprechenden α-Oximinoketonen der Formel

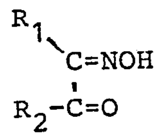

ringschließend kondensiert.

9. Verwendung der Triazolylcumarine gemäß Anspruch 1, als optische Aufheller, Scintillatoren und Laserfarbstoffe.

**Claims**

1. Triazolylcoumarins of the formula

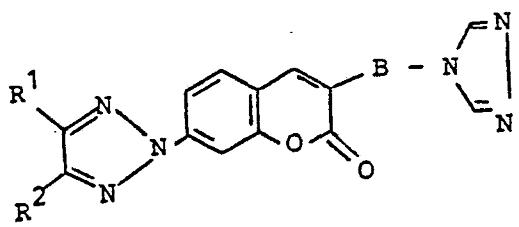

wherein

B denotes a direct bond or the 1,4-phenylene radical and

$R_1$ denotes $C_1$-$C_4$-alkyl,

$R_2$ denotes $C_1$-$C_4$-alkyl or phenyl which is optionally substituted by phenyl, $CH_3$, $OCH_3$ or Cl, or

$R_1$ and $R_2$ together form a fused benzene or naphthalene ring.

2. Triazolylcoumarins according to Claim 1, characterised in that

$R_1$ denotes methyl,

$R_2$ denotes ethyl, phenyl or 4-biphenylyl, or

$R_1$ and $R_2$ together form the missing members of a naphthalene ring linked *via* $C_1/C_2$.

3. Triazolylcoumarins according to Claim 1, characterised in that B represents a direct bond.

4. Triazolylcoumarin according to Claim 1, characterised in that

$R_1$ denotes methyl and

$R_2$ denotes phenyl.

5. Process for the preparation of triazolylcoumarins according to Claim 1, characterised in that salicylaldehydes of the formula

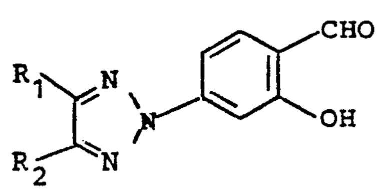

are condensed, in a manner which is in itself known, with triazole compounds of the formula

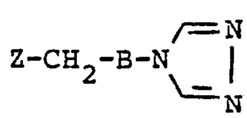

or the quaternisation products or protonation products thereof, wherein Z is an optionally functionally changed carboxyl group.

6. Process for the preparation of triazolylcoumarins according to Claim 1, characterised in that aminocoumarins of the formula

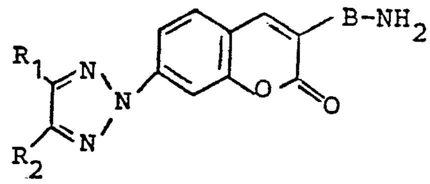

are reacted with amidrazones of the formula

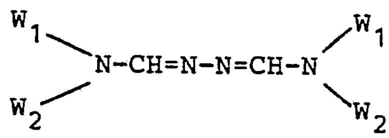

wherein

$W_1$ and $W_2$ denote $C_1$-$C_4$-alkyl or phenyl, or together denote $-(CH_2)_4-$ or $-(CH_2)_5-$,

and the reaction products are quaternised or protonated, if appropriate.

7. Process for the preparation of triazolylcoumarins according to Claim 1, characterised in that amidinocoumarins of the formula

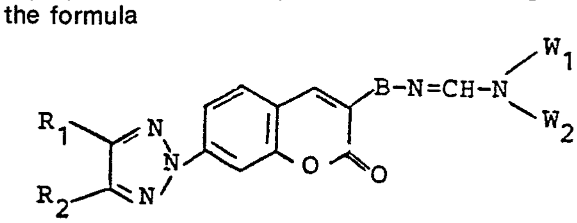

are reacted with formylhydrazine, and the reaction products are quaternised or protonated, if appropriate.

8. Process for the preparation of triazolylcoumarins according to Claim 1, characterised in that coumarins of the formula

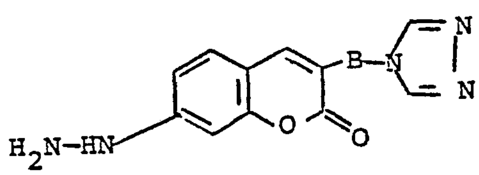

are condensed with appropriate α-oximinoketones of the formula

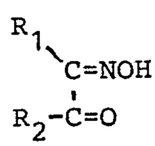

cyclisation taking place.

9. Use of the triazolylcoumarins according to Claim 1, as optical brighteners, scintillators and laser dyestuffs.

**Revendications**

1. Triazolylcoumarines de formule

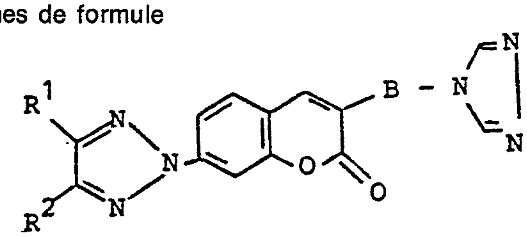

dans laquelle

B est une liaison directe ou le reste 1,4-phénylène et

$R_1$ est un reste alkyle en $C_1$ à $C_4$

$R_2$ est un reste alkyle en $C_1$ à $C_4$ ou un reste phényle éventuellement substitué par un radical phényle, $CH_3$, $OCH_3$ ou Cl, ou bien

$R_1$ et $R_2$ forment ensemble un noyau benzénique ou naphtalénique condensé.

2. Triazolylcoumarines suivant la revendication 1, caractérisées en ce que

$R_1$ est un groupe méthyle,

$R_2$ est un groupe éthyle, phényle ou 4-biphénylyle ou bien

$R_1$ et $R_2$ forment ensemble les chaînons manquants d'un noyau naphtalénique lié par les atomes $C_1/C_2$.

3. Triazolylcoumarines suivant la revendication 1, caractérisées en ce que B représente une liaison directe.

4. Triazolylcoumarine suivant la revendication 1, caractérisée en ce que

$R_1$ est le groupe méthyle et

$R_2$ est le groupe phényle.

5. Procédé de production de triazolylcoumarines suivant la revendication 1, caractérisé en ce qu'on condense d'une manière connue des salicylaldéhydes de formule

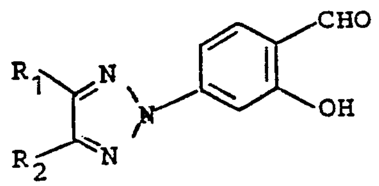

avec des composés triazoliques de formule

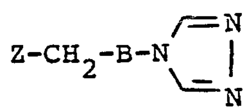

ou leurs produits de quaternisation ou de protonation, Z étant un groupe carboxyle éventuellement modifié du point de vue fonctionnel.

6. Procédé de production de triazolylcoumarines suivant la revendication 1, caractérisé en ce qu'on fait réagir des aminocoumarines de formule

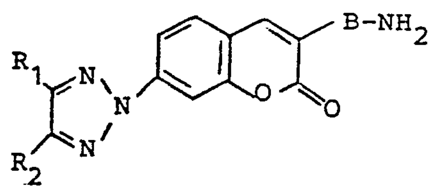

avec des amidrazones de formule

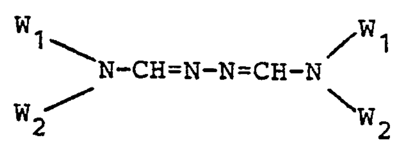

dans laquelle

$W_1$ et $W_2$ sont des groupes alkyle en $C_1$ à $C_4$, phényle, ou forment ensemble des groupes $-(CH_2)_4-$ ou $-(CH_2)_5-$

et on quaternise ou protone éventuellement les produits de réaction.

7. Procédé de production de triazolylcoumarines suivant la revendication 1, caractérisé en ce qu'on fait réagir des amidinocoumarines de formule

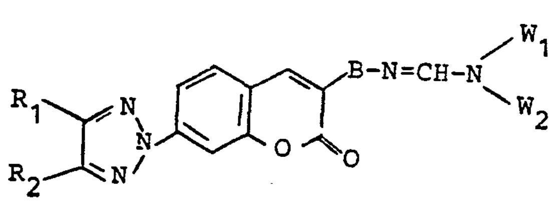

avec la formylhydrazine et on quaternise ou protone éventuellement les produits de réaction.

8. Procédé de production de triazolylcoumarines suivant la revendication 1, caractérisé en ce qu'on condense avec cyclisation des coumarines de formule

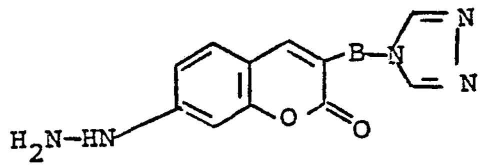

avec des α-oximinocétones correspondantes de formule

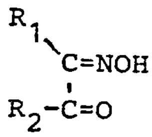

9. Utilisation des triazolylcoumarines suivant la revendication 1 comme azurants optiques, scintillateurs et colorants lasers.